# EUROPEAN PATENT SPECIFICATION

(11) **EP 4 122 391 B1**
(45) Date of publication and mention of the grant of the patent: **24.12.2025**
(21) Application number: 22460032.0
(22) Date of filing: 05.07.2022
(51) Int. Cl.: A61B 5/107, A61B 5/00, G01B 5/00, G01B 11/245, G03B 15/14, G03B 17/56

(54) **DEVICE FOR RECORDING CHANGES IN THE GEOMETRY OF THE HUMAN ABDOMINAL WALL USING THE PHOTOGRAMMETRIC TECHNIQUE**
VORRICHTUNG ZUR AUFZEICHNUNG VON VERÄNDERUNGEN DER GEOMETRIE DER MENSCHLICHEN BAUCHDECKE UNTER VERWENDUNG PHOTOGRAMMETRISCHER TECHNIK
DISPOSITIF D'ENREGISTREMENT DES CHANGEMENTS DANS LA GÉOMÉTRIE DE LA PAROI ABDOMINALE HUMAINE UTILISANT LA TECHNIQUE PHOTOGRAMMÉTRIQUE

(30) Priority: 22.07.2021 PL 43855521
(43) Date of publication of application: 25.01.2023
(73) Proprietor: Politechnika Gdanska, 80-233 Gdansk (PL)
(72) Inventor: Bielski, Pawel, 80-461 Gdansk (PL); Szepietowska, Katarzyna, 80-462 Gdansk (PL); Szymczak, Czeslaw, 80-414 Gdansk (PL); Lubowiecka, Izabela, 80-034 Gdansk (PL); Tomaszewska, Agnieszka, 82-300 Elblag (PL)
(74) Representative: Pawlowska-Bajerska, Justyna

(56) References cited:
- WO-A1-2015/108071
- US-A1- 2008 194 968
- US-A1- 2009 216 140
- US-A1- 2019 063 917

## Description

The invention relates to a device in the form of a station, which is placed over an individual lying down to record the geometry of their abdominal wall. The aim of the invention is to enable the effective collection of the photographic data needed to register this geometry using the photogrammetric technique. The results of measurements carried out by the invention are used in analyzes of the health condition of the human anterior abdominal wall and in research leading to the improvement of knowledge about the mechanics of the abdominal wall, which are the basis for the design of implants for the reconstruction of the abdominal wall, e.g. in the case of abdominal hernias and in postoperative hernia prevention.

So far the use of photogrammetric technique has been known in devices for registering the geometry of various objects - inanimate and alive.

From the patent description US10122997 a system is known for automatic generation of 3D geometry of inanimate objects based on a series of photos taken with a rotating stand in the shape of a fragment of a standing cylinder and a rotating table on which the object can be placed. The solution assumes the simultaneous use of several cameras. An example is given in which a cylindrical system is proposed, where the stand is next to the photographed object and the optical axes of the cameras are directed at the object located on the axis of rotational symmetry of the cylinder. The object and the stand are rotatable with respect to each other, with the axis of rotation being the axis of symmetry of the cylinder. Cameras can be moved along the height of the cylinder, and at the point of attachment they can also be rotated in a plane containing the height of the cylinder. However, this does not ensure that the cameras are evenly spaced from the tested object, unless the tested object also has a cylindrical geometry. In the case of measuring the geometry of e.g. abdominal walls in people of various shapes, e.g. obese people, it is an inconvenience because in this case the object is in the form of a hemisphere. An additional possibility of mounting the camera on a robotic arm has been described, but this does not ensure precise measurement of the geometry of objects with a small range of motion, such as the abdominal wall of a breathing human. It is also not possible to perform an examination of the anterior abdominal wall of a human lying on the back. Therefore, the invention cannot be used to register changes in the geometry of the human abdominal wall.

The description of the invention US2019166312 presents a system for generating 3D images of human skin, which uses cameras sensitive to UV light and cameras sensitive to the visible light range. The mounting of the cameras allows them to be rotated in relation to a person in a cylindrical system, where the axis of the cylinder is located along the spine. A set of cameras arranged in a cylindrical arrangement can be moved along the human body (spine). In the case of this solution, however, it is not possible to rotate the cameras so as to take into account the bi-curvature of the object, which we deal with in the case of some abdominal walls. The solution does not provide the possibility of an accurate reconstruction of the geometry of the abdominal wall in a supine position.

The description of the invention US2019063917 presents a multi-camera station for measuring the full, spatial geometry of a stationary inanimate object with the use of a camera stand in the form of a sphere. This solution requires the use of multiple cameras. It is not possible to place a person in the measurement field of this solution in a way that meets the requirements of examining the human abdominal wall in hospital conditions. From WO2015108071 a three dimensional data generation device, formed object production system is known. Subject is photographed from different directions using a plurality of cameras. The plurality of cameras are disposed roughly equidistantly from the subject such that the lines of sight thereof intersect at roughly the center of the subject. Of the plurality of cameras, a camera for photographing a surface having low roughness, and the like, also photographs an identification mark at the same time as the subject. A computer generates three-dimensional data on the basis of still image data obtained through photography using the plurality of cameras.

From US2009216140 an electric instrument used for the measurement of heart- and vascular function as well as body spaces, which is based on impedance measurement, the position of the electrodes, body volume and the volume of the segments located between the electrodes are measured with the help of a contactless measuring apparatus is known. The instrument comprises a plurality of electrodes capable of being attached to a body, a reference surface on which the body may be placed, and an electrical impedance meter capable of measuring the bodily functions of the body by measuring the electrical impedance and estimating the shape of the body by locating the electrodes attached to the body with reference to the reference surface, wherein the measured electrical impedance is associated with the estimated shape of the body segment.

Therefore, the aim of the invention was to provide a solution enabling the registration of the abdominal geometry of a living object - that is human being. This was achieved thanks to the appropriate development of the camera system on the device.

A device for recording the geometry of the human anterior abdominal wall using the photogrammetric technique was provided. This technique involves taking multiple photos of an object with the images overlapping each other. It is necessary to photograph the human abdominal wall in many shots, as it is more similar to a double-curvature surface than to a simple cylinder. Therefore, in order to reproduce the curvature in both directions, it was shown to take a set of photos in a double curvature system. The device does not automatically generate 3D models. The photographic data collected during the use of the measuring station requires processing and only then is the 3D model obtained.

The present invention is defined by the appended claims. More precisely, the present invention relates to a device for recording changes in the geometry of the abdominal wall of an object in the form of a human being using the photogrammetric technique comprising an arm in the form of a stand on which at least two cameras are mounted, and according to the invention, the device further comprising a plurality of brackets, revolute joints, two telescopic poles, and from 2 to 8 cameras, which are fixed in detachable and movable manner using said brackets on the arm in the shape of a half a dodecagon , placed on said telescopic poles by means of said revolute joints for positioning of the lenses in the line of the arch surrounding the abdominal wall of the object in the transverse direction. The registration of the geometry of this wall in the transverse plane while the angles α between its individual sections are min. 140 and max. 160 degrees, stably positioned by said two telescopic poles, wherein at all equal α angles, the arm is in the shape of a regular dodecagon part while at different α angles it is in the shape of a non-regular dodecagon part. Additionally the cameras are movably mounted in a way that enables the cameras to be rotated on the arm in the plane of the arm and in a plane perpendicular to the plane of the arm and perpendicular to the arm in the place where each camera is attached. The cameras are mounted on the arm at a distance from 15 cm to 50 cm from each other and the arm is movably and releasably attached by revolute joints to telescopic poles being detachably attached to the top of the poles in such a way that it is possible to rotate the arm by the angle β in the range of 360° in relation to the straight line connecting both revolute joints. The dimensions of the arm, adjustable height of the telescopic poles and using revolute joints allow to adjust the distance of the lens of each separate camera from the object during registration to be minimum 40 cm and maximum 65 cm, and additionally in the revolute joint a lock (E6) is made for blocking the rotation of the arm by the angle β minimum every 10 degrees and maximum every 30 degrees.

Preferably, the lock of the revolute joint (E5) is made using two disks with holes and a bolt with a nut.

Preferably, it comprises 4 cameras.

Preferably, all structural elements of the device are made of aluminum.

Preferably, the angles of the sections from which the arm is formed are equal.

### Detailed description of the invention

According to the invention, the cameras are mounted - fixed/attached on a arm in a form of a polygonal chain - in form of stand/trivet, placed on two telescopic poles by means of revolute joints, which ensures the positioning of the lenses in the line of the arch surrounding the abdominal wall of the subject in the transverse direction and the registration of the geometry of this wall in the transverse plane. At the same time, it is possible to rotate the arm by an β angle in the range of 360° in relation to the straight line connecting both ends of the arm thanks to the revolute joints on which the arm is mounted, which enables the required series of photos to be taken with the use of a limited number of cameras, i.e. from two to eight, - in the examples four. The combination of these two features makes it possible to collect photographic data for the photogrammetric representation of the geometry of the tested object in a two-curvature system, which has not been ensured by the solutions known so far. The photos are taken simultaneously with several, at least two cameras placed on the arm located above the abdominal wall, at most eight. The arm geometry is selected specifically for the geometry of the human abdominal wall subjected to various intra-abdominal pressure. The proposed solution enables the measurement of the geometry of double-curved objects, stationary or showing little movement in relation to the measuring station, such as the human abdominal walls. The measurement may be performed in a hospital setting using standard medical procedure to measure intra-abdominal pressure.

The handles can be attached to the arm, e.g. with screws, and then the cameras are attached to the brackets also with screws so that they are removably mounted on the arm. Fixing the cameras to the arm with the use of brackets ensures their even distance from the tested object and similar inclination angles of the cameras in relation to the surface tangent to the surface of the tested object, which allows to reduce distortions of geometry mapping and maintain a similar resolution of the obtained images of a given area. Cameras are mounted on the arm at a distance of 15 cm to 50 cm from each other, which depends on the number of cameras used in such a way that the more cameras, the smaller the distance between them, so as to ensure partial overlapping of adjacent shots, which is necessary in photogrammetric analysis. The adjustment of the position of the brackets on the arm is possible thanks to the use of oval holes in the arm, in which the brackets are mounted with the help of screws. The cameras do not have to be at the same distance, but at a similar distance. It is important that the cameras are attached at such a distance that the pictures taken - the shots - overlap.

The distance between the camera lenses and the photographed object is a minimum of 40 cm and a maximum of 65 cm thanks to technical features such as telescopic poles with adjustable height, the rotary arm and movable camera brackets. This is particularly important in the case of examining the human abdomen, even of a very convex shape. The short distance of the lenses from the object is necessary in hospital conditions, where it is not possible to set the measuring equipment at a considerable distance - the test is performed in a treatment room.

The rotary arm of the device is fixed on telescopic poles by means of revolute joints enabling the arm to be rotated by β angle in relation to the horizontal axis connecting both revolute joints. There is one such hinge on each of the two poles. Each pole is connected to a revolute joint and to the arm with bolts. One of the joints comprises a lock for the rotation of the arm at β angle every minimum 10 degrees and maximum 30 degrees, constructed using two discs with holes and a bolt and nut locking the joint in the desired position. Thanks to the blockade, the arm is rotated by a known angle β, i.e. every minimum 10 degrees and maximum 30 degrees, as many times as needed to perform a full reconstruction of the abdominal geometry. This makes it possible to measure the different geometries of the abdominal wall, even with a significant convexity. The arm is releasably attached to the telescopic poles, the cameras are releasably attached to the arm. The arm is movably mounted and the cameras are movably mounted.

The invention need to use little space in the consulting, medical room and does not disturb the work of medical personnel during working. The device is demountable, and therefore mobile - it can be easily and quickly moved, assembled and prepared for research in the hospital. The shape of the arm and its mounting on the poles with the use of revolute joints makes it possible to take a set of photos at the same time while maintaining constant angles of setting the focal lengths of the lenses in relation to each other and to the measured object, which also allows you to adjust the ranges of overlapping of the pictures taken. All this means that the solution enables the reconstruction of changes in the geometry of double-curved objects. The following series of photos are taken in a short time thanks to the use of radio triggers and the use of different positions of the rotary arm of the stand. The solution is tailored to meet the requirements resulting from the use of the photogrammetry method to reconstruct the geometry of the abdominal wall of a lying human. In addition, the solution is adapted to use in treatment rooms with limited space. The position does not limit the access of medical personnel to the patient. Additionally, there are the following advantages of using the device:
- Privided measuring system enables the collection of data suitable for the accurate reconstruction of the three-dimensional geometry of the abdominal wall.
- The measurement is non-contact, i.e. you do not touch the registered object - the patient during the test, which makes the measurement less burdensome for patients.
- Thanks to the use of a rotating arm with the use of joints of a stand with several cameras, which is rotatable thanks to the applied revolute joints, the procedure of collecting photographic data is fast, which translates into a short examination time. This is an important advantage of the device because: (i) during a medical procedure, the patient can only lie still for a short time; (ii) the test enables the measurement of the temporary abdominal wall geometry at a given stage of the medical procedure performed.
- Oval openings in the arm and articulated camera brackets make it possible to adjust the distance of the cameras and the orientation of their focal lengths relative to the photographed surface according to any geometry of the abdominal wall (more and less convex).
- Thanks to this construction and synchronous taking of pictures from several cameras, the problem of changing the geometry when breathing or other small movements of the patient is reduced.
- A small number of cameras can be used in the device.
- The device is small, does not require the use of electric wires, so it takes up little space in the treatment room and does not interfere with the work of medical personnel.
- The structure is stable and resistant to vibrations and accidental movement of the station during standard medical procedures, which ensures high accuracy of the measurement and, at the same time, the safety of the patient and the person performing the measurement.
- The structure can be dismantled, so it can be easily stored and transported. Individual elements are demountable by removing, for example, bolts from each connection.
- In view of the above, the stand is highly functional in hospital conditions.

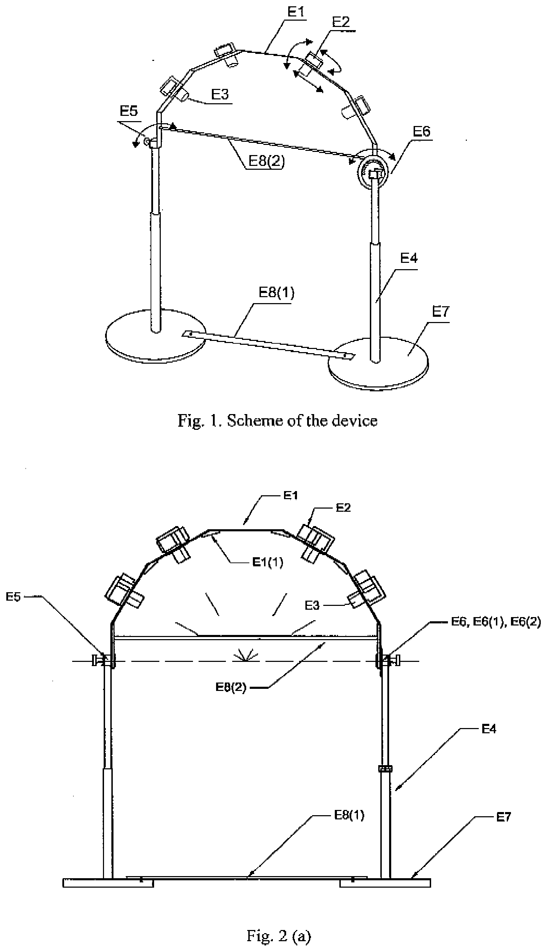

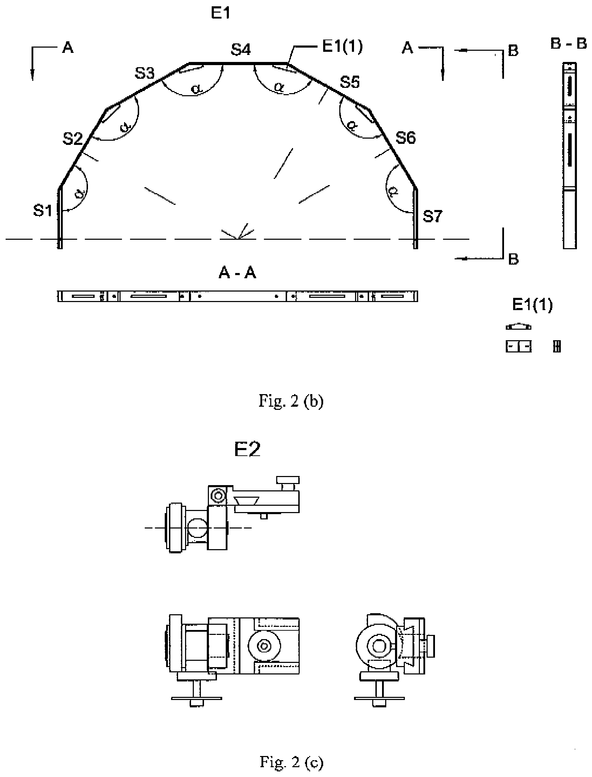

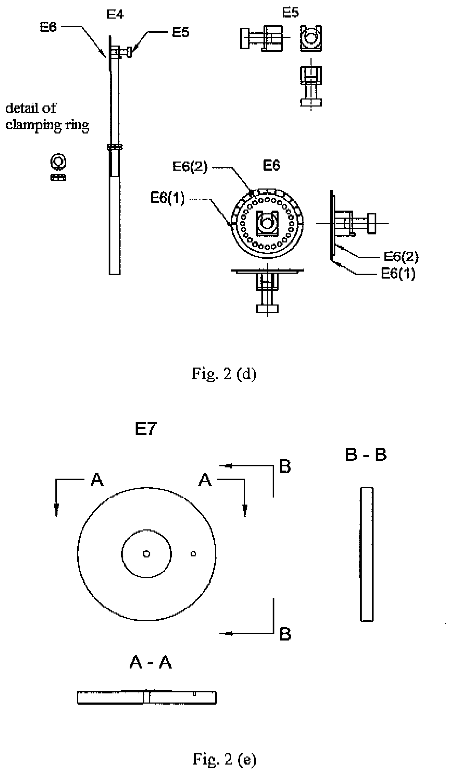

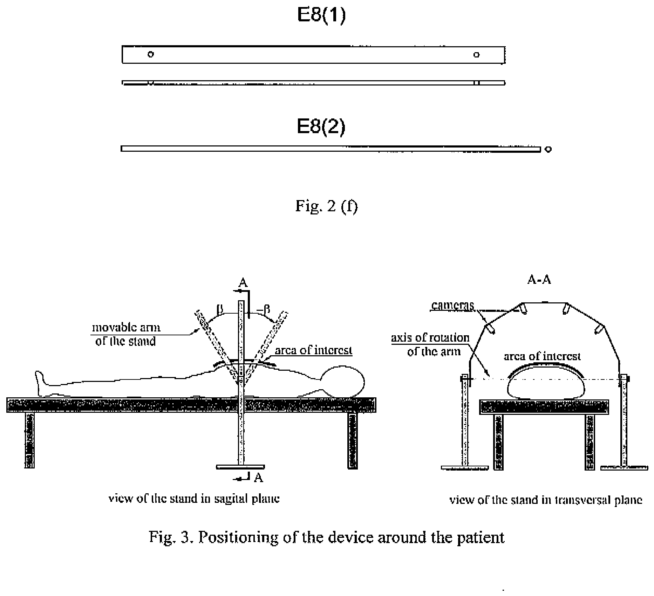

The invention is described in more detail in the embodiment and in the drawings, where in Fig. 1 the device is shown in an axonometric view and its components have been marked, in Figs. 2 a-f all structural elements of the device are shown in detail, and in Fig. 3, the position of the stand above the patient is shown. Fig. 2 shows details - a close-up of the device, i.e. (a) view of the device, (b) rotary arm E1 and E1(1) inserts, (c) handle E2, (d) telescopic pole E4, revolute joint E5 and lock E6, (e) E7 column base, (f) E8 (1) and E8 (2) bars

The device therefore comprises the most important components described below.

The rotary arm E1 composed of seven straight sections, forming a polygonal chain along the arc line. The turning angle α is 150 degrees in the example. The arm has oblong holes in which the E2 swivel brackets for E3 cameras are mounted with the help of screws.

E2 brackets for E3 cameras comprises two directional revolute joints. One joint enables rotation of the apparatus from the plane of the arm, in the direction perpendicular to the E1 arm in the place where the apparatus is attached, and the other in the plane of the arm. The E3 cameras are equipped with radio triggers and are located by means of a screw in the E2 brackets. The device contains from two to eight E3 cameras.

The E3 cameras are triggered by radio to take pictures synchronously.

Two E4 telescopic poles to support the E1 arm, variable height, to set the desired distance between the cameras and the photographed object.

Two joints - flat E5, fixed to the poles by means of bolts, to which the arm E1 is attached with bolts, enabling the device arm E1 to be rotated by an angle β in relation to the horizontal axis connecting both joints E5.

One rotation lock E6 of the disc-type joint E5, fixed to one of the joints E5, in the form of two disks with holes and a bolt and a nut.

Two disc-shaped E7 column bases for stable support of the poles.

Additionally, the device is stabilized and for this purpose connectors E8 (1) and E8 (2) are used. Connectors stabilize the device located at the test site during its assembly and are disassembled during measurements. The connectors are in the form of bars, of which the E8 connector (1) connects the two column bases to each other, and the other E8 (2) connects both ends of the rotary arm. Both connectors are connected to the structure with bolts.

The individual elements E1-E7 are removably attached to each other - in the examples connected to each other by means of screws.

### Example

### A) Construction of the device

As shown in Figs. 1 and 2, the device comprises such elements as: the arm E1 with the shape of a regular dodecagon part, four rotating brackets E2 for cameras, four cameras E3, two telescopic poles E4, two revolute joints with plain rotation possibility E5, one rotation lock type E6 disc joint E5 flat joint, two E7 disc-shaped column bases, two E8 (1) and E8 (2) connectors. The elements are connected to each other as follows. The E7 column bases are connected with the E8 connector (1) with bolts. Then, the E4 poles are placed on the bases with the help of screws. The E5 joints, the E6 lock and the E1 arm are mounted on the posts with the help of bolts. The arm is stabilized with the E8 connector (2), mounted with screws. On the arm, with the help of screws, the E2 camera brackets are mounted, on which the E3 cameras are mounted with the help of screws.

The details of the structure shown in Fig. 2 are described below:
The aluminum arm E1, shown in Fig. 2b, with a cross-section of 40x5 mm, in a shape of polygonal chain with vertices in six places at a distance of 0.2 m from the ends of the flat bar and at a distance of 0.32 m between successive vertices. The vertices of the polygonal chain made of flat bar divide it into 7 segments s1-s7 shown in Fig. 2b. Segments s2-s6 of E1 arm are inscribed in an arch with a span of 1.2 m and height 0.46 m, which is adapted to the average shape of the abdominal wall of an adult lying on their back. Both ends of the arm E1 have holes for the passage of the bolt fixing the columns E4, the joints E5 and the lock E6 (on one side), shown in Fig. 2d. The segments s2, s3, s5, s6 of the arm are drilled with oval holes 120x8 mm in size, for attaching the brackets E2, shown in Fig. 2c, to the E3 cameras. The oval shape of the holes allows you to set the distance between the cameras according to the size of the tested object. The inside angles of the arm α in this example measure 150 degrees between all the segments. Between the segments s2-s3, s3-s4, s4-s5 and s5-s6 there are inserts E1 (1), shown in Fig. 2b, made of aluminum, screwed to the arm by means of screws. E1 inserts (1) stiffen the arm structure. The individual elements E1-E7 are removably attached to each other by means of screws. In the example, the E7 column bases are connected by the E8 connector (1) with bolts. Then, the E4 poles are placed on the bases with the help of screws. The E5 joints, the E6 lock and the E1 arm are mounted on the poles with the help of bolts. The arm is stabilized with the E8 connector (2), mounted with screws. On the arm, with the help of screws, the E2 camera brackets are mounted, on which the E3 cameras are mounted with the help of screws.

Brackets E2 for E3 cameras, shown in Fig. 2c with adjustment of their orientation in two planes, 4 pieces. The design allows the E3 cameras to be rotated in the plane of the arm in the range of 360 degrees and around the arm segments in the range of 180 degrees. The E2 brackets are adapted to the standard screw mounting of the E3 cameras and are made of aluminum. This example uses 4 E3 cameras. The distance between the cameras is respectively 33 cm, 46 cm and 32 cm, while the distances of the camera lenses from the patient's abdomen are respectively 53 cm, 47 cm, 47 cm, 52 cm.

The poles of the device E4, shown in Fig. 2d, 2 pieces. The E4 poles are made of aluminum. The telescopic structure of the poles makes it possible to adjust the position of the stand arm above the patient. The height of the pole is adjustable in the range of 60-120 cm. The poles are locked in a given position with aluminum bolt clamps.

Joint E5, shown in Fig. 2d, enabling rotation of the arm of the device relative to the horizontal axis connecting the two joints, 2 pieces. The E5 joint is made of aluminum.

The lock E6 in the disc type with a mechanism for locking the arm of the device in a specific position corresponding to the selected value of the angle β, shown in Fig. 2d, 1 piece. The E6 lock is made of aluminum. The mechanism comprises of 2 parallel circular discs rotating about a common axis passing through their center. The larger E6 shield (1) is attached to the E1 arm and the smaller E6 (2) to the E4 pole. The 10° perforation of the discs makes it possible to block their mutual rotation every 10° by means of a bolt and a nut. The selected values of the β angle define the position of the E1 arm. The example uses the angle β in the range -30°: 30°, so seven positions of the E1 arm are used, each with an angle of β=10°.

E7 column bases, shown in Fig. 2e, 2 pieces. Round plates with a diameter of 405mm and a thickness of 35mm are equipped with 4 screw sockets for fixing the poles and one socket for the screw fixing the bottom bar E8 (1) in the base. The bases are made of aluminum, but they are heavy and ensure the stability of the structure.

Two connectors - the first lower connector E8 (1) attached to the bases E7 of the E4 columns and the second upper connector E8 (2) attached to the arm E1, stabilizing the device during its assembly, shown in Fig. 2f. The lower E8 connector (1) is made of an aluminum flat bar with a cross-section of 50x12mm and a length of 1075mm. At its ends, holes with a diameter of 13 mm are drilled through which the screw fixing the E8(1) connector is passed in the E7 base of the device. The E8(2) upper connector is made of a 15mm diameter aluminum bar that is screwed into the E1 arm with threaded ends. The upper connector E8(2) is always disassembled for the duration of the measurement, while the lower connector E8(1), depending on the needs, can remain installed or can be removed.

### B) Mode of operation - method of using the device (not part of the claimed invention)

As shown in Fig. 3, the measuring station should be positioned over the lying patient so that the center of the examined area of the abdominal wall lies on the axis of rotation of the arm E1, which is the stand for the cameras. For this purpose, it is necessary to adjust the height of the E4 telescopic poles. Then, you should mount the E3 cameras in the E2 brackets and orient them so that as much of the tested object as possible is visible in each frame. The axis of rotation of the arm should be in the center of the frame. After the cameras have been framed and focused, equip them with wireless radio triggers and start measuring. The measurement is based in taking simultaneous photos with the use of a wireless radio trigger. If necessary, it is possible to change the angle β of the E1 arm from the β=0 position to other positions differing from the previous one by an angle of ± 10° and to take additional series of photos in the new position. The angle β is changed by mutual rotation of the discs in the E6 lock. Repeat the operation until the desired number of photos is obtained. The data collected in this way is the basis for photogrammetric processing and reconstruction of the geometry of the tested object. The stand allows you to take pictures at various stages of the medical procedure. Thanks to this, it is possible to reconstruct the geometry of the abdominal wall in various states of deformation, and thus to register changes in the geometry.

After the procedure, the device is disassembled in such a way that the bolts securing the individual elements of the device to other elements are removed and other elements are detached from the device, in the following order: first the E3 cameras are removed, then the E2 brackets, then the arm E1, then the E4 poles together with the E5 joints and the E6 lock. Finally, the bases of the E7 poles remain, from which the lower E8 connector (1) is removed, if it was left for the duration of the test.

## Claims

1. Device for recording changes in the geometry of the abdominal wall of an object in the form of a human being using the photogrammetric technique comprising an arm in the form of a stand on which at least two cameras are mounted, the device further comprising a plurality of brackets, revolute joints, two telescopic poles, and from 2 to 8 cameras (E3), which are fixed in detachable and movable manner using said brackets (E2) on the arm (E1) in the shape of a half a dodecagon, placed on said telescopic poles (E4) by means of said revolute joints (E5) for positioning of the lenses in the line of the arch surrounding the abdominal wall of the object in the transverse direction and the registration of the geometry of this wall in the transverse plane while the angles α between its individual sections are min. 140 and max. 160 degrees, stably positioned by said two telescopic poles, wherein at all equal α angles, the arm is in the shape of a regular dodecagon part while at different α angles it is in the shape of a non-regular dodecagon part, and additionally the cameras (E3) are movably mounted in a way that enables the cameras to be rotated on the arm (E1) in the plane of the arm and in a plane perpendicular to the plane of the arm (E1) and perpendicular to the arm in the place where each camera is attached (E3), while the cameras (E3) are mounted on the arm (E1) at a distance from 15 cm to 50 cm from each other and the arm (E1) is movably and releasably attached by revolute joints (E5) to telescopic poles (E4) being detachably attached to the top of the poles (E4) in such a way that it is possible to rotate the arm (E1) by the angle β in the range of 360° in relation to the straight line connecting both revolute joints (E5), while the dimensions of the arm (E1), adjustable height of the telescopic poles (E4) and using revolute joints (E5) allow to adjust the distance of the lens of each separate camera (E3) from the object during registration to be minimum 40 cm and maximum 65 cm, and additionally in the revolute joint (E5) a lock (E6) is made for blocking the rotation of the arm (E1) by the angle β minimum every 10 degrees and maximum every 30 degrees.

2. The device according to claim 1, wherein the lock (E6) of the revolute joint (E5) is made using two disks with holes and a bolt with a nut.

3. The device according to claim 1, wherein it comprises 4 cameras (E3).

4. The device according to claim 1, wherein all structural elements of the device are made of aluminum.

5. The device according to claim 1, wherein the angles of the sections from which the arm (E1) is formed are equal.

## Patentansprüche

1. Vorrichtung zur Erfassung von Änderungen der Geometrie der Bauchdecke eines menschlichen Objekts mittels photogrammetrischer Technik, umfassen einem Arm in Form eines Stativs, an dem mindestens zwei Kameras montiert sind, sowie mehrere Halterungen, Drehgelenke, zwei Teleskopstangen und 2 bis 8 Kameras (E3), die mittels der Halterungen (E2) lösbar und beweglich am Arm (E1) in Form eines halben Zwölfecks befestigt sind, der mittels der Drehgelenke (E5) auf den Teleskopstangen (E4) platziert ist, um die Linsen in der Linie des Bogens zu positionieren, der die Bauchdecke des Objekts in Querrichtung umgibt, und die Geometrie dieser Decke in der Querebene zu erfassen, wobei die Winkel α zwischen ihren einzelnen Abschnitten mindestens 140 und höchstens 160 Grad betragen, stabil positioniert durch die beiden Teleskopstangen, wobei der Arm bei allen gleichen α-Winkeln die Form eines regelmäßigen Zwölfecks hat, während er bei unterschiedlichen α -Winkeln die Form eines unregelmäßigen Zwölfecks hat, und zusätzlich sind die Kameras (E3) beweglich montiert, so dass die Kamera am Arm (E1) in der Ebene des Arms und in einer Ebene senkrecht zur Ebene des Arms (E1) und senkrecht zum Arm an der Stelle gedreht werden kann, an der jede Kamera (E3) befestigt ist, während die Kameras (E3) in einem Abstand von 15 cm bis 50 cm voneinander am Arm (E1) montiert sind und der Arm (E1) ist durch Drehgelenke (E5) beweglich und lösbar an Teleskopstangen (E4) befestigt, die abnehmbar an der Spitze der Stangen (E4) so angebracht sind, dass es möglich ist, den Arm (E1) um den Winkel β im Bereich von 360° in Bezug auf die gerade Linie zu drehen, die die beiden Drehgelenke (E5) verbindet, während die Abmessungen des Arms (E1), die einstellbare Höhe der Teleskopstangen (E4) und die Verwendung der Drehgelenke (E5) es ermöglichen, den Abstand der Linse jeder einzelnen Kamera (E3) vom Objekt während der Registrierung auf mindestens 40 cm und höchstens 65 cm einzustellen, und zusätzlich ist im Drehgelenk (E5) eine Sperre (E6) vorgesehen, um die Drehung des Arms (E1) um den Winkel β mindestens alle 10 Grad und höchstens alle 30 Grad zu blockieren.

2. Vorrichtung nach Ansprüche 1, wobei, dass die Verriegelung (E6) des Drehgelenks (E5) durch zwei Scheiben mit Löchern und eine Schraube mit Mutter erfolgt.

3. Vorrichtung nach Anspruch 1, wobei, dass sie 4 Kameras (E3) umfasst.

4. Vorrichtung nach Ansprüche 1, wobei alle Strukturelemente der Vorrichtung aus Aluminium bestehen.

5. Vorrichtung nach Ansprüche 1, wobei die Winkel der Abschnitte, aus denen der Arm (E1) gebildet ist, gleich sind.

## Revendications

1. Dispositif d'enregistrement des variations de la géométrie de la paroi abdominale d'un objet en forme d'être humain, par une technique photogrammétrique, comprenant un bras en forme de support sur lequel sont montées au moins deux caméras. Le dispositif comprend également plusieurs supports (E2), des articulations rotoïdes, deux perches télescopiques et de 2 à 8 caméras (E3), fixées de manière à pouvoir être détachées et mobiles au moyen desdits supports (E2) sur le bras (E1) en forme de demi-dodécagone, placé sur les perches télescopiques (E4) au moyen des articulations rotoïdes (E5) pour le positionnement des lentilles dans l'axe de l'arc entourant la paroi abdominale de l'objet dans le sens transversal et l'enregistrement de la géométrie de cette paroi dans le plan transversal, les angles α entre ses sections individuelles étant compris entre 140 et 160 degrés, positionné de manière stable par lesdits deux poteaux télescopiques, dans lequel à tous les angles α égaux, le bras a la forme d'une partie dodécagonale régulière tandis qu'à différents angles α, il a la forme d'une partie dodécagonale non régulière, et de plus les caméras (E3) sont montées de manière mobile de manière qui permitte à la caméra d'être tournée sur le bras (E1) dans le plan du bras et dans un plan perpendiculaire au plan du bras (E1) et perpendiculaire au bras à l'endroit où chaque caméra est fixée (E3), tandis que les caméras (E3) sont montées sur le bras (E1) à une distance de 15 cm à 50 cm l'une de l'autre et le bras (E1) est fixé de manière mobile et amovible par des articulations rotoïdes (E5) à des poteaux télescopiques (E4) qui sont fixés de manière amovible au sommet des poteaux (E4) de telle sorte qu'il est possible de faire tourner le bras (E1) de l'angle β dans une plage de 360° par rapport à la ligne droite reliant les deux articulations rotoïdes (E5), tandis que les dimensions du bras (E1), la hauteur réglable des perches télescopiques (E4) et l'utilisation d'articulations rotoïdes (E5) permettent de régler la distance entre l'objectif de chaque caméra (E3) et l'objet indépendamment pendant l'enregistrement entre un minimum de 40 cm et un maximum de 65 cm, et de plus dans l'articulation rotoïde (E5) un verrou (E6) est réalisé pour bloquer la rotation du bras (E1) par l'angle β. Ceci entre minimum tous les 10 degrés et maximum tous les 30 degrés.

2. Dispositif selon la revendication 1, dans lequel le verrouillage (E6) de l'articulation rotoïde (E5) est réalisé à l'aide de deux disques percés et d'un boulon avec écrou.

3. Dispositif selon la revendication 1, **caractérisé en ce qu'**il comprend 4 caméras (E3).

4. Dispositif selon la revendication 1, dans lequel tous les éléments structurels du dispositif sont en aluminium.

5. Dispositif selon la revendication 1, dans lequel les angles des sections à partir desquelles le bras (E1) est formé sont égaux.
